(19) Europäisches Patentamt
European Patent Office
Office européen des brevets

(11) **EP 4 190 245 A1**

(12) **EUROPEAN PATENT APPLICATION**

(43) Date of publication:
**07.06.2023 Bulletin 2023/23**

(21) Application number: **21212452.3**

(22) Date of filing: **06.12.2021**

(51) International Patent Classification (IPC):
**A61B 8/08** (2006.01)  **A61B 6/00** (2006.01)

(52) Cooperative Patent Classification (CPC):
**A61B 6/5294;** A61B 6/5217

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventor: **BRAUNAGEL, Andre**
**Eindhoven (NL)**

(74) Representative: **Philips Intellectual Property & Standards**
**High Tech Campus 52**
**5656 AG Eindhoven (NL)**

(54) **QUANTITATIVE RADIOGRAPHIC IMAGING USING A 3D CAMERA**

(57)   System (SYS) and related method for image processing. The system is configured to process X-ray projection imagery ($\lambda$) acquired of an object (PAT) by a medical X-ray acquisition module (XAM), based on thickness data (z) acquired of the object by a non-ionizing thickness data collector (TDC). The system allows for quantitative imaging for a quantity of interest such as attenuation coefficient.

**FIG. 1**

EP 4 190 245 A1

**Description**

FIELD OF THE INVENTION

The invention relates to a system for image processing, an imaging arrangement, a computer-implemented method, a computer readable medium, and a computer program element.

BACKGROUND OF THE INVENTION

Whilst X-ray radiography is the most common of all medical imaging modalities, it has certain disadvantages. One of the shortcomings is a lack of quantitative imaging capability. Radiography is based on projection imagery. The imaging setup is for acquiring information on the attenuation of X-rays as they pass through patient tissue. The attenuation is then registered in terms of intensity loss. The underlying attenuation mechanism may be described by the Lambert-Beer law. But since the thickness of the object/tissue imaged is not known, estimations of the linear attention coefficient based on the initial intensity and the measured intensities alone is not normally possible.
As a consequence, for example in chest X-ray imaging, it may be at times difficult for a radiologist to differentiate between a patient of low/normal BMI (body mass index), who might have some abnormalities in the lung that could explain an increased level of attenuation, and a high(er)-BMI-patient having a normal lung, as the measured intensities may approximately be the same.

SUMMARY OF THE INVENTION

There may be a need for improved imaging for certain quantities of interest, such as in attenuation-based imaging, in particular in relation to X-ray imaging.
An objective of the present invention is achieved by the subject matter of the independent claims where further embodiments are incorporated in the dependent claims. It should be noted that the following described aspect of the invention equally applies to the imaging arrangement, to the method, to the computer program element and to the computer readable medium.
According to a first aspect of the invention there is provided a system for image processing, configured to process X-ray projection imagery acquired of an object by a medical X-ray acquisition module, based on thickness data acquired of the object by a non-ionizing thickness data collector.
In embodiments the processing yields X-ray quantity of interest in relation to the object, wherein the said X-ray quantity of interest includes at least any one of more of i) attenuation coefficient, ii) diffusion coefficient, iii) gradient of electron density.
The X-ray quantity may be any other such quantity that is line integrable along propagation rays of X-radiation.
In embodiments the system of claim is configured to visualize on a display device a map of said X-ray quantity of interest. The map includes spatially resolved distribution of the quantity of interest in relation to the imaged object.
In embodiments the thickness data is acquired along a predefined direction relative to the optical axis of the X-ray acquisition module. The optical axis may be defined as an (imaginary) line run between X-ray source of the module and an X-ray sensitive detector of the module. The X-ray sensitive detector converts received X-ray radiation, after interaction with the object, into the projection X-ray imagery.
In embodiments, the thickness data so acquired is normalized by system against base line data. The base line data represent spatial depth data relative to the detector when the object is not present between the X-ray source and the detector. Base line data may be acquired in a calibration phase or operation. The thickness data represents such depth data, but with the object or parts thereof present between X-ray detector and X-ray source.
In embodiments the processing by the system includes spatially registering the X-ray projection imagery and the thickness data relative to each other.
In embodiments the processing by system takes prior anatomical knowledge on material types for the object into account. Based on anatomical knowledge, such as an anatomical model or atlas, sections of in-tissue path length through material type not of interest can be excluded, whilst retaining path length thorough material(s) that are of interest. For example, path lengths through bone and/or fat may be excluded, but lung tissue path length is retained to better relate the quantity of interest to lung tissue, a quantity of interest in chest X-ray imaging.
In embodiments the system is configured to processes the X-ray quantity of interest into diagnostics information. The diagnostics information may relate for example to a diagnostic prediction of osteoporosis.
In embodiments, the thickness data collector includes any one of more of: i) a depth sensing camera, ii) an infrared camera, iii) a laser-based camera, iv) an optical camera, v) an ultrasound measurement device, vi) a radar measurement device.
In another aspect there is provided an imaging arrangement, including a system of any one of the above-mentioned embodiments, and including the X-ray acquisition module and/or the thickness data collector.

In embodiments, the thickness data collector is arranged on or at the X-ray acquisition module or has any other pre-defined location with respect to the acquisition module.

In embodiments the medical X-ray acquisition module is one of any one of i) a radiography apparatus, ii) a mammography imaging apparatus, iii) a bone density X-ray system, vi) a tomosynthesis system.

In another aspect there is provided a computer implemented image processing method, comprising: processing X-ray projection imagery acquired of an object by a medical X-ray acquisition module, based on thickness data acquired of the object by a non-ionizing thickness data collector.

In another aspect there is provided a computer program element, which, when being executed by at least one processing unit, is adapted to cause the processing unit to perform the method.

In another aspect there is provided at least one computer readable medium having stored thereon the program element.

The proposed system may be used in particular with an X-ray imaging apparatus that includes, in addition to its X-ray acquisition component, one or more 3D cameras. Previously, such cameras were introduced in order to assist an operator of the X-ray imaging apparatus. For example, such integrated 3D camera may help detect landmarks of the patient and may assist in adjusting collimation automatically for example. Whilst the use of such 3D cameras for adjusting pre-scan parameters is recognized, it is proposed herein to put such or similar cameras to new and beneficial use: specifically, it is proposed herein to use the spatial (depth) information provided by such cameras for image post-processing to achieve new quantitative X-ray imaging (as opposed to mere qualitative X-ray imaging as currently done) for linear attenuation coefficients (LAC) for example. Combining information from the X-ray imagery and from the 3D camera as proposed herein yields enhanced diagnostic information. What is proposed herein is to extract quantitative information from X-ray projection imagery, such as the LACs or other quantities of interest. With the proposed system, the LAC can be imaged directly ("quantitatively"), thus enhancing diagnostic accuracy. Bone density imaging may be done based on the proposed system, previously requiring dedicated X-ray imaging equipment such as dual energy imaging equipment.

However, the system is not confined for use with X-ray system with such extant integrated 3D camera. The system allows for cost effective retrofitting of existing, generic, general purpose radiography apparatuses to convey new functionalities, such as the bone density measurement capabilities and others that rely on linear attenuation coefficient estimation.

The system may also be used in radiation therapy planning for example.

"Object" is used herein in the general sense to include animate "objects" such as a human or animal patient or anatomic parts thereof, but also includes inanimate objects such as an item of baggage in security checks or a product in non-destructive testing. However, the proposed system will be discussed herein with main reference to the medical field, so we will be referring to the "object" as "the patient" or a part of the patient, such as an anatomy or organ, or group of anatomies or organs of the patient, etc.

BRIEF DESCRIPTION OF THE DRAWINGS

Exemplary embodiments of the invention will now be described with reference to the following drawings, which, unless stated otherwise, are not to scale, wherein:

Fig. 1 shows a schematic block diagram of an X-ray imaging arrangement;
Fig. 2 shows data processing system for quantitative imaging;
Fig. 3 shows a calibration operation for acquiring reference depth measurements;
Fig. 4 shows facilitations to support depth measurement calibration; and
Fig. 5 shows a flow chart of a computer-implemented data processing method for quantitative imaging.

DETAILED DESCRIPTION OF EMBODIMENTS

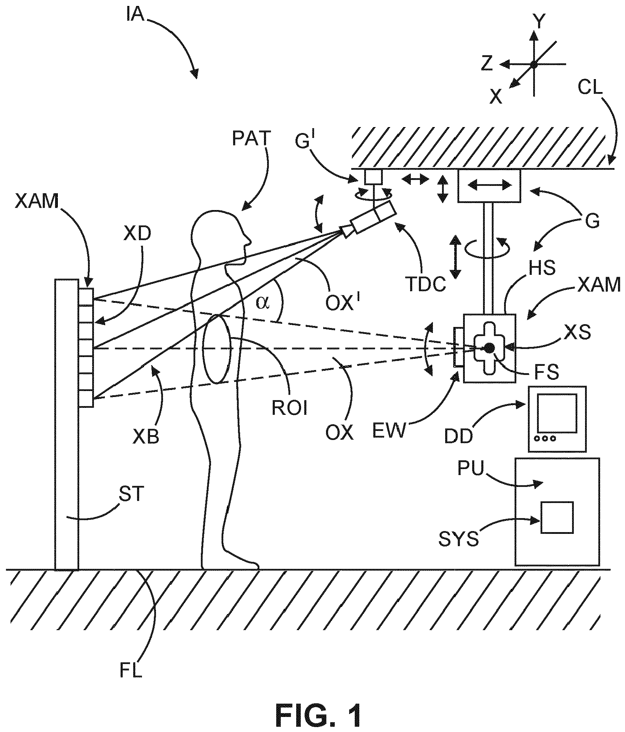

Reference is now made to the schematic block diagram of Fig. 1 which shows components of an X-ray imaging arrangement IAR.

The arrangement IAR may include an X-ray imaging apparatus IA configured to acquire projection imagery of a region of interest ROI in a patient PAT. The arrangement may further include a data processing unit PU, such as one or more computer systems, configured to receive the projection imagery for processing, storing or visualizing as required. For example, the data processing unit(s) PU may run a visualizer VIZ that allows rendering visualizations of the acquired projection data for visual representation in a graphics display on a display device DD.

The arrangement IAR is generally configured for attenuation-based X-ray imaging. However, the arrangement may include an image processing system SYS that enables instead or in addition quantitative rather than mere qualitative attenuation-based imaging. As will be explored in more detail below, the image processing system SYS allows imaging for spatially resolved (linear) attenuation coefficients of the region of interest, rather than for mere relative intensity attenuation, although that later can still be done herein in addition if required. The image processing system SYS may be arranged in hardware or software, or partly in both. In embodiments, the data processing system is implemented by the one or more computing units/system PU. The system may be integrated into the imaging apparatus IA. The system may be integrated into an operator console or a workstation part of, other otherwise associated with, the imaging apparatus IA. Before explaining operation of the image processing system SYS in more detail, reference is made first to imaging apparatus.

The X-ray imaging apparatus IA may be of the fixed installation type as shown in Fig. 1, but mobile solutions are also envisaged where a component of a system is mounted on a wheeled or a tracked dolly that can be spatially moved to where it is needed, so its use is not confined to a specific examination room as shown in Fig. 1.

The X-ray imaging apparatus is configured for radiography such as in chest X-ray imaging but imaging of other body parts is also envisaged herein. Attenuation based projection imaging is mainly envisaged herein, however, this is not at the exclusion of other imaging modalities such as spectral imaging, or dark field, or phase contrast imaging. Diagnostic type X-ray imagers are envisaged herein, enabled for example for bone density measurements or mammography, etc. Other types of X-ray imaging apparatus, not necessarily of the diagnostic type, such of the interventional type are also envisaged herein. One example of interventional imagers include C-arm imagers.

Broadly, the imaging apparatus may include an X-ray acquisition module XAM. Acquisition module XAM includes an X-ray source XS and an X-ray sensitive detector XD. The X-ray source may be mounted in a housing HS including an egress window EW through which, upon energization, an X-ray beam XB issues forth from a focal spot FS towards the region of interest. X-radiation interacts with matter/tissue in the region interest ROI, such with lung tissue, bone etc. The so interacting beam traverses the region of interest is then detected as a set of intensities at X-ray sensitive pixels $X_j$ of the detector XD arranged behind the patient PAT/ROI. There is thus an examination region, a portion of space, defined between source XS and the X-ray sensitive detector XD in which the patient PAT, or at least the region of interest, resides during imaging. The detector is preferably of the flat panel type. The X-ray sensitive pixels $X_j$ are arranged in 2D to form an X-ray sensitive surface.

Acquisition circuitry (not shown) may include an A/D conversion component that converts the detected intensities into digital values. The digital values may then be made available as 2D projection imagery. The 2D imagery comprises spatially resolved digital values, storable as a matrix (rows and columns) of corresponding digital values. The spatially resolved digital values are thus the (2D) projection image. One or more such projection images may be acquired.

By wired or wireless communication interfacing, the projection image may be forwarded to the processing unit PU for processing, storing or other, as described above. In particular, the image processing system SYS may process the projection image(s) so forwarded. The system SYS may instead be integrated into the imaging apparatus itself in some embodiments.

The X-ray source XS, in its housing HS, may be mounted in a gantry G that allows a spatial propagation direction of the X-ray beam to be adjusted. The beam XB may thus travel along optical axis OX in different spatial directions so as best to irradiate the region of interest. For example, pitch, roll, yaw may be adjusted manually or by actuators. The different degrees of freedom are indicated in Fig. 1 by double arrows. Not all degrees of freedoms are illustrated in Fig. 1, and more or less such degrees of freedom are envisaged herein. A collimator (not shown) may be arranged in the housing HS for example, to adjust a width of the beam XB. For a given collimation aperture, orientation of the optical axis OX defines the field of view ("FOV") of the X-ray acquisition module XAM. This field of view may be designated herein as FOVx. The X-ray source's optical axis OX can be thought of as an imaginary line extending from the focal spot FS of the X-ray source to a pre-defined point, such as a mid-point, of the radiation sensitive surface of the X-ray detector XD.

In a ceiling CL mounted arrangement as shown in Fig. 1, the gantry G may include a movable coupling system such as a railing or roller system integrated into the ceiling. Such a coupling may allow moving the X-ray source in a plane parallel to the ceiling to substantially any desired location in the examination room. A fixture instead of such a movable coupling system may be used instead. However, such a ceiling mounted option is merely according to one embodiment and is not mandatory herein. Instead of the ceiling mounted solution, a wall-mounted or floor mounted solution may be envisaged. As shown, the X-ray apparatus is of the unfettered type as there is no (permanent) physical coupling between the X-ray source XS and the detector XD. However, this is also not mandatory herein as alternative options are also envisaged, such as in C-arm imaging or others, where the X-ray source and the X-ray detector are mounted opposite each other in an X-ray gantry (eg, the said C-arm).

In the unfettered type, the X-ray source XD may be mounted on a floor mounted stand ST as shown in Fig. 1. However, wall mounted options are also envisaged herein as an alternative. The X-ray detector may likewise be mounted so that it can assume different poses to facilitate alignment with the X-ray source. Fixed mounting options for the X-ray detector are also envisaged. In mobile imaging solutions, the detector is a standalone module that is placed behind the patient (relative to the imaging dolly) for imaging.

During imaging, the patient in the examination region may lie on an examination table. However, the patient may also stand or squat or assume any other suitable position or pose as required. Before imaging, the FOVx is adjusted, to so that the ROI, such as patient's chest, is within the X-ray beam.

As opposed to the imaging set-up envisaged herein, previous approaches may be confined to mere qualitative X-ray imaging. The modelling assumption for matter versus X-ray interaction underlying attenuation-based X-ray imaging may follow the Beer-Lambert law whish posits:

$$I = I_0 \cdot exp(- \int \mu(x)dx) \qquad (1)$$

wherein, $I_0$ is the initial intensity, and $\mu(x)$ is the attenuation coefficient.

Qualitative, as opposed to quantitative X-ray imaging, merely allows imaging for the relative intensity (ratio) between incoming and detected intensities $I$, $I_0$, respectively. Rearranging terms in (1), it is the logarithm of this intensity ratio that is used for displaying purposes, whilst it is the outgoing intensity $I$ and incoming intensity $I_o$ that are measured at the detector (the latter in a calibration setup). It is this log-ratio that confers image contrast. Whilst such qualitative imaging may indeed serve useful purposes and is envisaged herein in some embodiments as an add-on, what is proposed herein as a departure from mere qualitative imaging is an additional, novel image processing system SYS. The envisaged image processing system SYS allows quantitative imaging. The system is configured to resolve the measured intensities into values that may directly represent the quantity of interest, in particular, the linear attenuation co-efficient $\mu$ or related such coefficients that describe intrinsic matter versus X-radiation interaction.

Such quantitative imaging is enabled herein by having a thickness detector component TDC operate in conjunction and coordination with the X-ray acquisition module XAM. The thickness detector DDC may be implemented as an optical camera with its own field of view and optical axis OX'. The camera TDC may be arranged to acquire an optical image of the region of interest. In particular, the camera TDC allows acquiring an image using non-ionization radiation of the region of interest relative to the imaged plane of the X-ray detector. Instead of an optical camera, a (native) depth sensing camera may be used. A range of camera technologies are envisaged herein, such as infrared (IR), near infrared (NIR), laser based (such as LIDAR), radar, ultrasound, or any other imaging technology based on non-ionizing radiation principles. In the following, the spatial data acquired by camera TDC will be referred to herein as depth sensing map or depth sensing data, whichever camera technology is used. The depth sensing data may be native raw data as acquired, or, as may be the case for example for optical imagery, may require some post-processing to compute the depth sensing maps from the acquired raw spatial data. The depth sensing measurements relate to in-tissue path length or thickness, and may thus be also referred to herein as thickness measurements.

The depth sensing information/map acquired by the camera TDC is such that it allows ascertaining a spatially resolved thickness of the patient at the ROI along the optical axis OX of the X-ray imager IA. From this thickness/in-tissue path length, the LAC may be computed (see eq (2) below). Registration may be required or the two field views of camera and X-ray imager module XAM are in native registry. More than one camera TDCi positioned spatially apart relative to the ROI, operable to acquire multiple depth sensing maps along different directions /optical axes OX'$_i$ are envisaged herein in embodiments. Triangulation may be used to ascertain the true patient thickness at the ROI from the set of multiple depth sensing maps relative(/along the X-ray optical axis OX.

In the representation of Fig. 1, for clarity, there is an exaggerated distance shown between the region of interest and the detector XD. In reality, the patient PAT would be expected to assume a position close to the (outer) proximal surface of the detector (that is the one closer to the X-ray source). For example, in some instances, the patient may be as close so as to touch with the body part to be imaged (such as the chest) the X-ray's detector proximal surface.

The camera TDC is configured to produce depth sensing information. Depth sensing information, denoted herein as $z$, allows measuring the through tissue path length and based on it extracting the linear attenuation co-efficient according to the following computation based on eq (1):

$$\mu = - ln\left(\frac{I}{I_0}\right) \cdot \frac{1}{z} \qquad (2)$$

Preferably, the camera is mounted at a known, predefined location/pose, so that the mutual spatial constellation between the camera TDC and the X-ray source is a priori known. The camera TDC may have its own gantry G' and may be, as shown, ceiling mounted, floor mounted, wall-mounted or mounted in any other arrangements. The camera may be arranged on the X-ray apparatus itself. For example, the camera may be mounted on the gantry G of the X-ray source or indeed on the housing of the X-ray source, on the collimator (not shown) or on any other suitable location at or in the X-ray imager.

The known mutual spatial constellation of the camera TDC and the X-ray source XS allows to pointwise correlate the depth measurements and the X-ray projection imagery. Preferably some or each measured intensity is correlated with a respective depth measurement that quantifies thickness or in tissue- path length at that point where the correlated intensity was measured by detector XD. Facilitated by camera TDC, the depth map may be obtained. The depth sensing map may be (spatially) registered on the projection image. Calculation routine (2) may be applied for example per pixel to convert the originally acquired intensity-attenuation based X-ray projection image into a quantitative image of the spatial distribution of the LACs.

In one embodiment, the known spatial constellation is such that the two optical axes OX' and OX are essential coinciding to achieve native registry. A collimation function of the camera may be used to adjust the width of the depth sensing map to that of the X-ray image. Alternatively, pre-processor PP at the camera TDC, or as included in the system SYS may crop the depth sensing map accordingly so as to adjust its width to that of the X-ray image. For example, the camera may be mounted on the egress window on the collimator in a position in front of the focal spot FS. The circuitry and hardware of the camera TDC can be configured to be of very low X-ray opacity so that the loss of X-ray intensity as beam XB passes through the camera TDC's circuitry/hardware is negligible.

The gantry or mounting mechanism G' of the in-tissue path length finding camera TDC may allow the camera to assume, similar to the X-ray source, a number of degrees pose and/or shifts to facilitate aligning the two FOVs.

Instead of mounting the camera TDC so that the two optical axes are permanently coinciding as mentioned, it may be sufficient in some embodiments that the two axis momentarily coincide as the camera TDC and/or the X-source XS is/are moved repeatedly and in synchrony. For example, the camera TDC may be moved through its gantry or other positioning mechanisms into the field of view of the X-ray source when this is off, and can be placed in front of the focal spot on the optical axis OX of the X-ray imager to acquire depth sensing image. Before the X-ray image is acquired, the camera is moved out of the FOVx. Alternatively, the camera is moved into the FOVx after X-ray acquisition. This synchronization of the two acquisitions (X-ray and depth sensing) can be done manually by user, or by a control logic CL automatically. The control logic CL may address a set of actuators to effect the movement of the camera TDC or X-ray source XS. Thus, coordination/control logic CL for coordinating acquisition may enable not only simultaneous, but also sequential imagining by X-ray module XAM and camera TDC, in whichever order.

The camera TDC acquires two sets of the depth sensing imagery $z, z_0$ in two modes or operational phases. Thus, the camera is operable in two modes, calibration mode and in a patient imaging mode. In calibration mode, depth sensing measurement of the detector surface XD is acquired with no patient present in the camera's FOV. Thus, patient is not occluding the detector surface XD. In patient mode, depth sensing data is acquired with the patient in front of the detector present. The detector surface is now at least partly occluded by the body part of interest. Patient mode may be run for example prior or after X-ray image acquisition for protocol efficiency.

The set of reference/baseline calibration depth measurement data $z_0$ acquired in calibration mode and the set of depth measurement $z$ acquired during a patient mode may be processed by the image processing system SYS. The system SYS allows extraction (eq (2) ) of the quantity of interest, in particular of the linear attenuation co-efficient to so enable (direct) quantitative imaging as described above.

A single such pair of depth sensing data $z, z_0$ is sufficient if there is no or merely negligible patient movement. The same sets of depth measurement may be used to convert not only one but plural X-ray images into LAC imagery. A motion detector arrangement may be used. If patient motion is detected, the depth measurement acquisition is rerun. The depth sensing camera itself may be used in such a motion monitoring mode, or another dedicated motion detector system is used instead, not necessarily image-based.

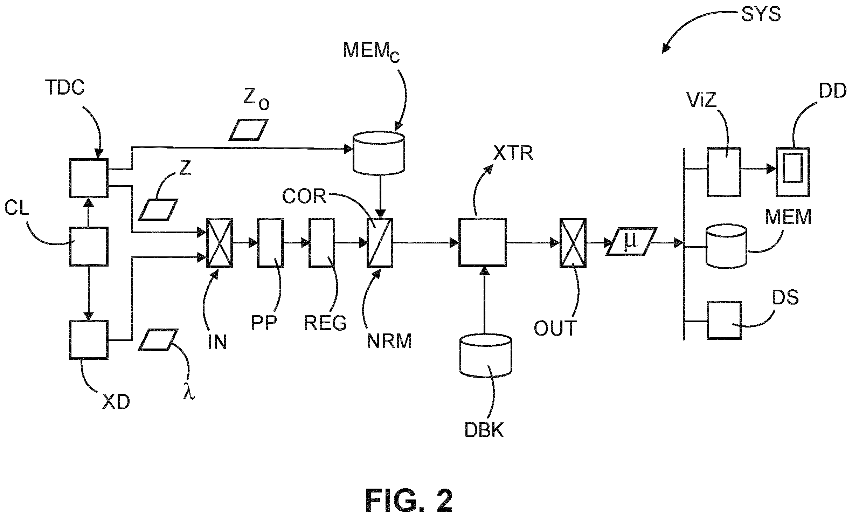

Operation of the quantitative imaging component system SYS is now explained in more detail with particular reference to the schematic block diagram in Fig. 2.

Depth sensing measurements including the calibration data $z_0$ and the patient depth measurement $z$ are received at one or more interfaces IN.

The calibration data calibration data $z_0$ may be acquired by camera TDC in a previous calibration session. The calibration data may be stored in a calibration memory MEMc. Acquisition of calibration data may be done once, but can be done repeatedly to accommodate different circumstances as will be explained in more detail below.

What is also received is the projection imagery $\lambda$ acquired by the X-ray detector XD. The depth sensing measurements $z$, $z_0$ may be acquired before or after X-ray image. The patient depth sensing data and the calibration data are not necessarily acquired in immediate sequence, although this may still be done.

Optional pre-processing circuitry PP such as a cropper (for cropping depth sensing data), noise filter, or other conditioners may be used to process the depth sensing measurements ($z, z_0$) and/or the projection imagery $\lambda$. Optionally, the pre-processing is done onsite at the camera TDC or at the X-ray imager.

Optionally, if the field of view or optical axes of the camera and the X-ray module are not coinciding, a registration component REG is used to spatially register the depth sensing maps and the X-ray projection imagery.

Normalizer component NML is used to normalize the patient depth sensing measurement $z$ based on a normalizer function $f$, using the calibration/ baseline depth measurements z0 to estimate the patient thickness map. For example, in one embodiment, the patient depth sensing measurement is subtracted from the baseline depth sensing measurement z0 so that the actual in-tissue path length through the patient can be calculated.

Extractor XRT may combine pixel or region-wise the normalized depth measurement $z'=f(z,z0)$ with the projection measurements $\lambda$ based on a combiner function $C(z',\lambda)$, such as according to routine eq(2), by multiplication or division. For example, the LAC may be extracted as per $\mu_i = C(z',\lambda) = \lambda_i/z_{i'}$, with index "$i$" indicating pixel position. The LAC map $\mu_i$ may then be output though interface OUT. The LAC map, as one example of quantitative imaging, with its values indicative of the respective spatially applicable linear attenuation co-efficient, or other quantity of interest.

The attenuation co-efficient map $\mu$ may then be made available for processing, storage in memory, or visualization by visualizer VIZ on a display device as required. The map $\mu_i$ may be visualized concurrently or in sequence with the X-ray projection image, other side by side, or one as an overly over, with suitable transparency. Optionally, the LAC map $\mu_i$ may be processed by a diagnostic system DS, for example, to assist in osteoporosis diagnosis or any other diagnostic task that relies on a pixelwise quantitative estimate of the quantity of interest, such the LAC. Other quantities of interest extractible herein may include attenuation coefficient, absorption coefficient, scattering coefficient, mass attenuation coefficient, mass absorption coefficient, mass scattering coefficient, logarithmic (Naperian or Decadic, or any other basis) attenuation coefficient, spectral hemispherical attenuation, directional attenuation coefficient.

Referring now in more detail to the registration module REG, as mentioned this may be optional if the camera system and the X-ray source are spatially arranged in native registry to that their optical axes OX, OX' are aligned in parallel or are indeed coincident at least at time during the respective acquisitions if such native registry is not possible a spatial constellation may arise as is shown in the example of Fig. 1, where there is a deviation $\alpha$ between the imaging axis OX, OX', a geometrical-spatial registration algorithm may be used. For example, in the situation depicted in Fig. 1, the depth measurements Z may be projected onto the X-ray optical axis OX to compute the aligned measurements that allow estimating the in-tissue path length along the X-ray source's optical axis OX. Triangulation may be used with depth measurement from multiple cameras $TDC_i$ *(i≥2, eg, i=3,* or other) arranged at different locations relative to the ROI. Alternatively, a single or some such camera(s) TDCi is/are moved through their gantries to different locations for different depth sensing acquisitions.

Several refinements are envisaged herein for the extractor XTR. For example, the linear attenuation co-efficient in the modelling assumption in Fig. 1 is simplified as there is an additional dependency on the X-ray energy and the material/tissue type or density. Different materials or the same material at different densities attract different linear attenuation coefficients.

So as to account for the material/density dependency, a modeling assumption may be made according to which the ROI is assumed to be made up of a hypothetical homogeneous materials or density. This simplified assumption may then yield an idealized linear attenuation co-efficient that represents the average of the linear attenuation coefficients of the materials that make up the region of interest in reality.

However, for a yet more refined materially resolved embodiment, anatomical prior knowledge may be used as stored in a knowledge database DBK. For example, one or more anatomical 3D models, such as a solid mesh-model or voxel model, may be held in memory DBK. Each model voxel codes for a material type or density. A bank of such anatomical models may be held as a function of patient bio-characteristics such as age, BMI, sex, etc. System SYS may include a user interface where patient data is received. In response thereto, system SYS queries model database DBK for the appropriate model given the patient data of current patient. Such models allow estimating from prior knowledge, per-projection direction, a rough estimate of the composition of different materials such as fat, bone, etc encountered on a given ray. The estimated path length may be divided into sections per material type. The above-described processing may then be done for a given material section of interest, disregarding the occluded and/or occluding sections for materials not of interest. The section of the occluding and or occluded materials may be subtracted from depth sensing measurement z', and it is this material-corrected length z* that is then used in (2) to calculate the LAC or related quantity: $z^* = z' - \Sigma_m z_m$, where index $m$ runs through material types not of interest on the given ray.

Since the organs typically overlay each other, further processing may be used to calculate the attenuation coefficient for different organs separately. In the example of chest imaging, in order to calculate the attenuation coefficient of for lung tissue, the materials of no-interest may include fat layer and bone (the ribs) to leave air and lung tissue of materials of interest. The fat and layer contribution may be subtracted by making for example an assumption that the thickness of the layer is approximately constant on the sides and the front and back of the patient. The thickness on the sides can be directly estimated from the X-ray image. In order to eliminate rib/bone contribution, any one of known subtraction algorithms may be used in addition to isolate the signal of interest (lung tissue and air) for improved quantification. This pro-processing prior to extraction may be done by a corrector COR. The corrector COR may also account for other impeding effects by applying suitable correction data c as will be described in more detail below. The described approach is not only interesting for chest imaging but for many other applications, like bone imaging, where this approach could be used to calculate the attenuation coefficient of the bones and thus reveal the risk for osteoporosis. Such bone density measurements were previously possible only with dedicated X-ray systems. The proposed system thus allows, among other benefits, cost effective retrofitting of existing generic X-ray imagers to perform bone density measurements.

In relation to the energy dependency, the proposed system may be used with spectral imaging, for example, in dual energy or photon-counting radiography. Source-enabled or detector enabled spectral imaging techniques are envisaged. In this manner in spectral imaging, multi-dimensional data is acquired with an additional energy dependency. For example, projection imagery in different energy channels such as high and low (or at three or more levels) are acquired and the proposed system SYS may operate separately as described above on some or each energy channel to obtain an LAC map per energy level $\mu^E$. The energy dependency refinement may be combined with the material sensitive processing based on medical knowledge database described above.

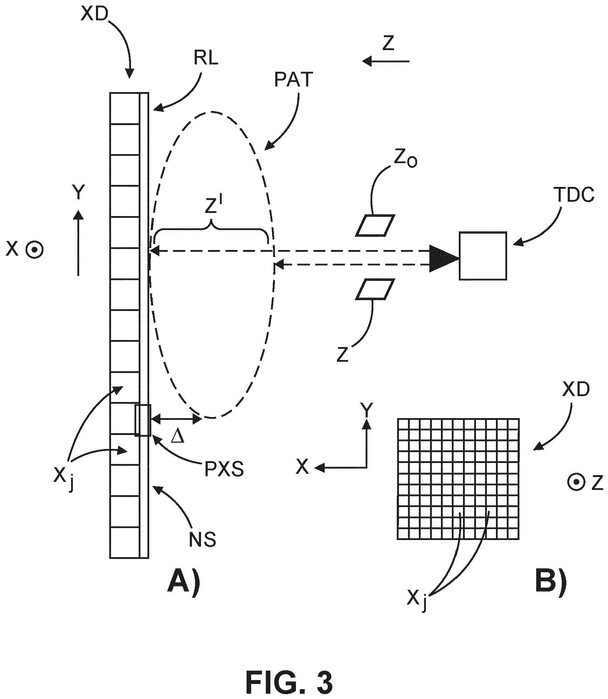

Reference is now made to Fig. 3 which illustrates the two modes of operation, calibration mode and patient mode, of camera TDC. In general, as in the previous Figures, a coordinate system is used where a Z direction indicates the direction of the optical axis OX of the X-ray system whilst, (X, Y) are the two dimensions of the preferably 2D image plane of the detector XD.

Fig. 3A shows a side elevation view of detector XD with patient PAT in front, along direction X extending into plane of the drawing. Fig. 3B affords a frontal view of the detector plane along the optical axis OX on the matrix of the X-ray sensitive surface formed by detector pixels Xj.

Fig. 3A illustrates operation of the two modes, with depth sensing measurement $z_0$ forming baseline or reference data acquired in calibration mode whilst the patient is not residing in the examination region in front of the X-ray detector. Before or after this calibration mode, the patient is then asked to assume a position in front of the detector XD similar to the one during imaging and the patient depth sensing data Z can then be acquired in patient mode. Patient mode operation may be run during X-ray imaging, or may be done beforehand or thereafter, as required.

As can be seen, the calibration depth measurements $z_0$ measure the distance between the sensor surface of the camera (not shown) TDC and the surface of the detector XD, whilst patient measurements z measure the distance from the sensor of the camera to the outer surface of the patient. The depth measurements in either mode are spatial data. For each pixel location Xj, the difference between the measurement of $z_0$ and $z_0$ may be calculated to obtain an estimate z' for the in-tissue path length L can be obtained.

The depth measurements, and in particular the patient depth measurements z, are likely to return good result if the patient body part in which the ROI is embedded, abuts against an outer layer, the receiving layer RL, of the detector module XS. The respective body part is thus preferably in close proximity to X-ray sensitive pixels Xj during imaging. This receiving layer RL (shown in side-elevation view of Fig. 3A) may be formed of a low opacity material such as plastic or other suitable material, preferably sufficiently resistant to abrasion and other impact to protect the underlying detector circuitry. The distance between outer layer RL and the detector pixel is a design quantity and can be factored in by normalizer NRL as a general design offset. Preferably, there are no gaps or lacunae Δ between the X-ray detector and the patient PAT at the region of interest during imaging. However, this may not always be achievable given characteristics of patient body structures or the imaging protocols to be used, and such gap(s) Δ may nevertheless emerge, whether or not wanted, as shown in Fig. 3. As can be appreciated from the geometry as sketched in Fig. 3, such patient versus detector XD gaps Δ may lead to spurious in-tissue path length estimates as computed by normalizer NRL.

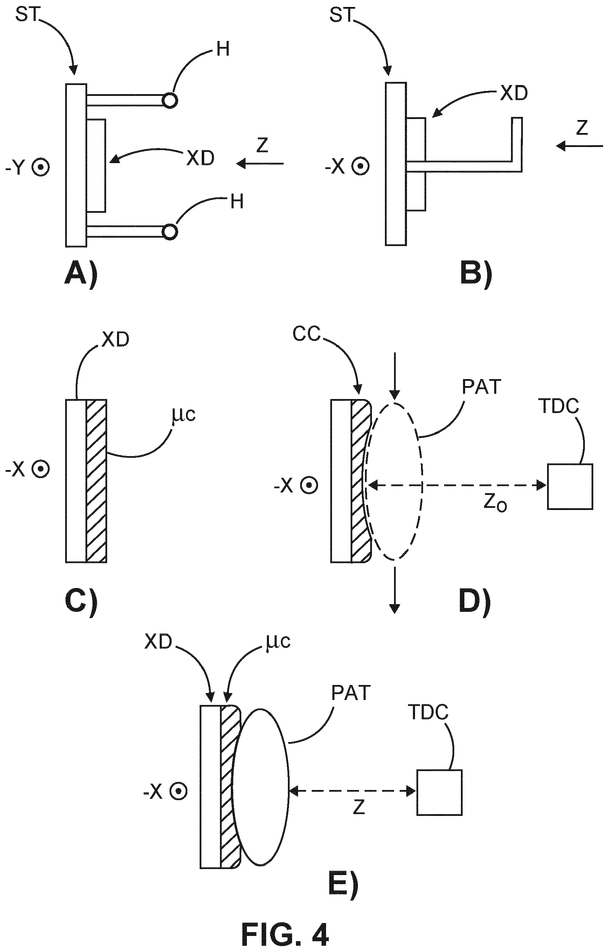

So as to improve the quality of depth sensing measurements by reducing likelihood of such gaps to emerge, various facilitations may be envisaged herein as schematically shown in Fig. 4. For example, Fig. 4A,B illustrates in plan view (4A) and side elevation (4B), a stand ST on which the detector XD is mounted, with cantilevered handlebars H projecting outwardly, for example perpendicular to the surface of the X-ray detector XD. The handlebars are arranged with the detector XD inbetween. The patient, when imaged by the camera TDC in patient mode, may be asked to firmly grab those handles and push their back or respective body parts flush against the surface of the detector against the receiving layer RL for example. The handle H or support may be formed in numerous ways and it may be understood that Figs. 4A,B is an example envisaged in embodiments. The arrangement H should be such that the patient can generate a force perpendicular to the X-ray sensitive surface of detector XD, so as to press or urge into contact the respective body part with the X-ray surface. Gaps Δ may thus be eliminated or at least minimized. Alternatively, or in addition, the handles HL may be arranged behind the detector for chest imaging from the back, with the patient using the handles to urge their chest into contact with the detector surface. On the upper edge of the detector housing, a recess may be provided for resting patients chin, as optional additional measure for encouraging patient to assume a position so as to contact the detector surface.

Another facilitation is shown in Figs. 4C-E to which reference is now made. In this embodiment, the receiving layer RL may be formed by a deformable molding component MC, such as cushion layer for example as illustrated in Fig. 4C. This can be fashioned from a layer of viscoelastic material which can be easily deformed, but remains for a certain period in the deformed state before relaxing back into its original shape prior deformation. A mold of the patient surface silhouette can be taken, if patient PAT is urging the relevant body part into contract with the molding component MC, thus exerting a certain pressure so that mold component assumes shape of a negative imprint of the patient's distal (away from the source) body surface.

Once the mold is shaped, the patient is asked to remove themselves, and camera TDC is operative to acquire depth sensing measurements of the depth profile to so acquire accurate base line depth measurements in calibration mode. Shortly after molding, the patient is then asked to move back into the same position so that the respective body part is now in registry with the mold, thus eliminating or at least reducing, any gaps Δ. This protocol using the mold MC is illustrated in Figs. 4D-E, with Fig. 4D showing no patient present after moldtaking with acquisition of mold profile zo, whilst Fig. 4E is the situation where the patient is present in the mold, ready for X-ray imaging and acquisition of patient depth sensing measurement z. The cushioned setup may be used in particular for mammography.

For some imaging protocols, or for some patients or for whatever reason, such gaps are wanted or cannot be avoided. For example, in some embodiments and imaging protocol, the patient should not abut the detector module XD. In such cases, a second set of "reverse" depth sensing measurements is acquired to probe the distance between detector surface and distal body surface (the one facing the detector and distal to source XD) of the patient. In the present context, this second set of depth sensing measurements may be referred to herein as proximity measurements. The proximity measurements thus measure the distance between distal patient body surface, whilst the patient depth sensing measurements z measure the distance between camera TDC sensor and patient's proximal body surface.

In some embodiments, the proximity measurements may be collected by a system of proximity sensors PXS. Sensors PXS may be included in the detector module XD such as in the receiving layer RL. The layout of the sensors PXS may be in the form of a matrix in 2D. The sensors PXS measure the local proximity to patient PAT's distal body surface. The proximity values may thus form another set of correction data that may be subtracted (in addition to the calibration data z0) from the corresponding depth measurements z collected in patient mode by camera TDC, to so yet more accurately estimate the true in tissue path lengths. Types of proximity sensors envisaged herein include those of the capacitance type, infrared, or others. Some such types of proximity sensor may be manufactured from components (circuity, etc) with very low X-ray opacity so can thus be mounted on top the X-ray pixels with merely negligible intensity loss. The proximity sensor data is preferably collected as spatial data, in pixelwise registry with depth sensing measurements. However, sometimes it may be sufficient to collect proximity measurements at a coarser resolution than the depth sensing measurement resolution and to use interpolation to relate proximity measurements to each pixel in the depth sensing map Z. A similar interpolation scheme may also be used in relation to the depth sensing measurements Z and the X-ray imagery. A lower resolution depth map may then be interpolated onto the X-ray image pixel layout of higher resolution for example, or vice versa if the depth sensing map has a higher resolution that the X-ray image λ.

The different kinds of correction data mentioned above, may be applied by a corrector COR. Operation of normalizer NRM and corrector COR may be done be combined into single component. Normalization and correction may be applied in any order prior to extraction.

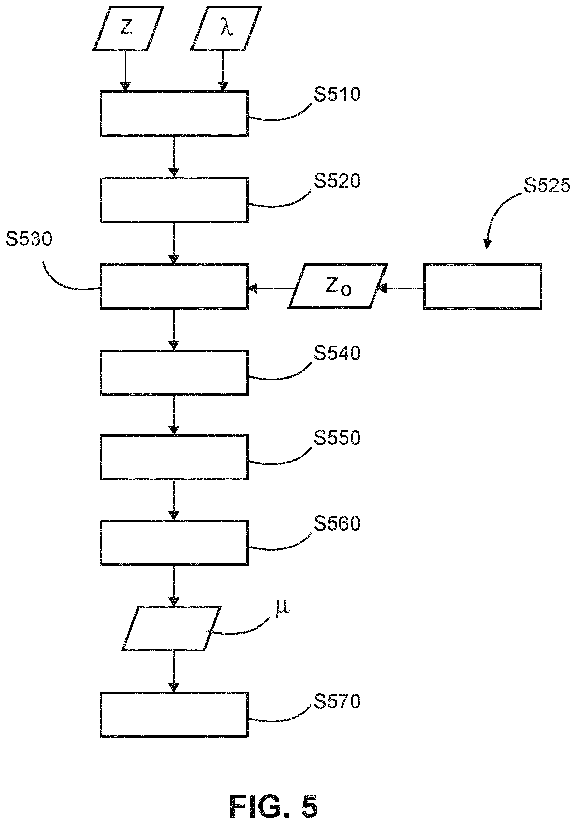

Reference is now made to Fig. 5 which shows a flow chart of a computer-implemented method as envisaged herein for quantitative attenuation imaging. Broadly, the method includes extraction of linear attenuation coefficients or other quantities of interest from attenuation imagery such as acquired in X-ray imaging, or other modality. In the following, main reference may be made to X-ray imaging, cognizant that other attenuation based imaging modalities are not excluded herein.

At step S510, depth sensing measurements z acquired of a patient at an X-ray detector of an X-ray imaging apparatus is received, and so is projection imagery λ acquired by the X-ray detector. The two spatial data sets may be acquired concurrently or in sequence. The two data sets z,λ may or may not be received at the same time.

At optional step S520 the two sets of spatial data, the depth sensing measurement (map) z and the X-ray image, are registered.

At step S530 a normalization operation is carried out based on calibration or baseline depth measurement data z0. The baseline depth measurement data z0 is acquired S525 at a different time than the depth sensing measurements z. The baseline depth measurement data z0 is acquired whilst the patient (or the body part of interest) is not at the X-ray detector. The calibration data may be arithmetically combined with the patient depth measurement data z to obtain normalized data z'. For example, the normalization operation may include subtraction z' = z-z0. The depth measurement data or the normalized data z' is an estimate for spatially resolved in-tissue path length for the body part X-ray imaged or to be X-ray imaged. The normalization step with an explicit calibration data set is optional as some depth measurement techniques do not rely on this, such a depth sensing with structured light or others. In some such embodiments, the depth sensing data for the patent and the baseline is acquired in the same acquisition. For example, the detectors outer surface RL may be integrated flush into a surrounding bezel surface of the mount stand ST or the detector is integrated into a wall for example with receiving surface RL flush with the wall surface, etc. The FOV of the depth sensing camera may then be chosen larger than the FOVX to acquires the whole depth sensing set z,z0 in a single acquisition. Depth measurements in relation to the non-occluded detector surface NS (see Fig. 3) or surface surrounding the detector (bezel, wall etc) may then represent implicit calibration data. Thus, patient mode and calibration mode are combined into as little as a single acquisition. Depth measurements in relation to the non-occluded detector surface NS may be identified as maximal values in the depth measurement set. The mentioned flushness is not a necessity herein, so long as the offset of the detector surface against a reference surface co-depth measured is pre-defined.

In addition, in an optional step S540, a correction may be applied to the depth measurements z or normalized depth measurements. The correction operation may include any one of more of i) the above-mentioned offset, ii) material specific path length sections for materials that are not of interest, and as may be derived from anatomical models in a database DBK, and iii) proximity data to eliminate patient versus detector gaps $\Delta$, as explained above.

The order of the optional registration, optional normalization and optional correction step is immaterial. Normalization can be done beforehand in a prepossessing step so that the measurements received at step S510 can be assumed to be already normalized. The correction operation S540 and the normalization step may be applied at once in a single step or operation $z' = F(z, z_0, c)$, with $F$ a function, and $c$ some or all of the correction data.

At step S550 a conversion routine is applied to extract LAC or related quantity of interest. X-ray measurements $\lambda$ are combined with the depth measurements, optionally normalized and/or registered. The conversion routine may be based on the Lambert-Beer model eq (2). For example, the attenuation values may be divided by the normalized depth measurements to obtain a map of the quantity of interest, such as a LAC map.

At step S560 the map of spatially resolved linear attenuation co-efficient is made available for additional processing at step S570 if required.

Said additional processing S570 may include visualization on a graphics display device DD and/or may include using a diagnostic algorithm to diagnose or detect diseases or conditions based on the estimates of the linear attenuation co-efficient or on a related one or more quantity.

Other X-ray quantities of interest for which the proposed system and method is applicable include diffusion coefficient for dark field imaging and electron density gradient for phase contrast. Both quantities, diffusion coefficient and electron density gradient, can be modelled as line integrable quantities, similar to the Beer-Lambert setup eq (1) above. For spectral imaging, the quantity of interest (attenuation coefficients, diffusion coefficient, electron density) may be obtained separately for different energies.

The components of the system SYS may be implemented as one or more software modules, run on one or more general-purpose processing units PU such as a workstation associated with the imager, or on a server computer associated with a group of imagers.

Alternatively, some or all components of the system SYS may be arranged in hardware such as a suitably programmed microcontroller or microprocessor, such an FPGA (field-programmable-gate-array) or as a hardwired IC chip, an application specific integrated circuitry (ASIC), integrated into the imaging system SYS. In a further embodiment still, the system SYS may be implemented in both, partly in software and partly in hardware.

The different components of system SYS may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described system. The computing unit can be adapted to operate automatically. The system may respond to user requests and execute the method accordingly. A computer program may be loaded into a working memory of a data processor. The data processor may thus be equipped to carry out the method of the invention.

This exemplary embodiment of the invention covers both, a computer program that right from the beginning uses the invention and a computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope. Such reference signs may be comprised of numbers, of letters or any of alphanumeric combination.

## Claims

1. System (SYS) for image processing, configured to process X-ray projection imagery ($\lambda$) acquired of an object (PAT) by a medical X-ray acquisition module (XAM), based on thickness data ($z$) acquired of the object by a non-ionizing thickness data collector (TDC).

2. System of claim 1, wherein the processing yields X-ray quantity of interest in relation to the object (PAT), wherein the said X-ray quantity of interest includes at least any one of more of i) attenuation coefficient, ii) diffusion coefficient, iii) gradient of electron density.

3. System of claim 1 or 2, configured to visualize on a display device (DD) a map of said X-ray quantity of interest.

4. System of any one of the previous claims, the thickness data so acquired along a predefined direction relative to the optical axis of the X-ray acquisition module (XAM).

5. System of any one of the previous claims, wherein the thickness data so acquired is normalized against base line data.

6. System of any one of the previous claims, wherein the processing includes spatially registering the X-ray projection imagery ($\lambda$) and the thickness data relative to each other.

7. System of any one of the previous claims, wherein the processing takes prior anatomical knowledge on material types for the object into account.

8. System of any one of the previous claims, configured to processes the X-ray quantity of interest into diagnostics information.

9. System of any one the previous claims, wherein the thickness data collector (TDC) includes any one of more of: i) a depth sensing camera, ii) an infrared camera, iii) a laser-based camera, iv) an optical camera, v) an ultrasound measurement device, vi) a radar measurement device.

10. Imaging arrangement (IA), including a system of any one of the previous claims, and including the X-ray acquisition module (XAM) and/or the thickness data collector (TDC).

11. Arrangement of claim 10, wherein the thickness data collector (TDC) is arranged on or at the X-ray acquisition module (XAM) or has any other pre-defined location with respect to the acquisition module (XAM).

12. Arrangement of any one of the previous claims, wherein the medical X-ray acquisition module (XAM) is one of any one of i) a radiography apparatus, ii) a mammography imaging apparatus, iii) a bone density X-ray system, vi) a tomosynthesis system.

13. Computer implemented image processing method, comprising: processing (S530) X-ray projection imagery ($\lambda$) acquired of an object (PAT) by a medical X-ray acquisition module (XAM), based on thickness data (z) acquired of the object by a non-ionizing thickness data collector (TDC).

14. A computer program element, which, when being executed by at least one processing unit (PU), is adapted to cause the processing unit (PU) to perform the method of claims 13.

15. At least one computer readable medium having stored thereon the program element of claim 14.

**FIG. 1**

**FIG. 2**

**FIG. 3**

**FIG. 4**

**FIG. 5**

Europäisches
Patentamt

European
Patent Office

Office européen
des brevets

## EUROPEAN SEARCH REPORT

Application Number

**EP 21 21 2452**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/250824 A1 (YI JAEMOCK [KR] ET AL) 6 August 2020 (2020-08-06) * paragraphs [0059], [0172], [0136], [0179], [0196] – [0198], [0209]; figures 3,17 * | 1-15 | INV. A61B8/08 A61B6/00 |
| X | US 2019/357844 A1 (RAUPACH RAINER [DE] ET AL) 28 November 2019 (2019-11-28) * paragraph [0009] * | 1-15 | |
| X | US 2015/222875 A1 (FERNÁNDEZ GARCIA AVELINA [ES] ET AL) 6 August 2015 (2015-08-06) * paragraph [0042] * | 1-15 | |

TECHNICAL FIELDS
SEARCHED       (IPC)

**A61B**

The present search report has been drawn up for all claims

| Place of search | Date of completion of the search | Examiner |
|---|---|---|
| Munich | 20 April 2022 | Koprinarov, Ivaylo |

EPO FORM 1503 03.82 (P04C01)

## ANNEX TO THE EUROPEAN SEARCH REPORT
## ON EUROPEAN PATENT APPLICATION NO.

EP 21 21 2452

This annex lists the patent family members relating to the patent documents cited in the above-mentioned European search report.
The members are as contained in the European Patent Office EDP file on
The European Patent Office is in no way liable for these particulars which are merely given for the purpose of information.

20-04-2022

| Patent document cited in search report | | Publication date | Patent family member(s) | | Publication date |
|---|---|---|---|---|---|
| US 2020250824 | A1 | 06-08-2020 | CN | 111513737 A | 11-08-2020 |
| | | | EP | 3689241 A1 | 05-08-2020 |
| | | | KR | 20200095859 A | 11-08-2020 |
| | | | US | 2020250824 A1 | 06-08-2020 |
| US 2019357844 | A1 | 28-11-2019 | CN | 110522466 A | 03-12-2019 |
| | | | EP | 3571997 A1 | 27-11-2019 |
| | | | US | 2019357844 A1 | 28-11-2019 |
| US 2015222875 | A1 | 06-08-2015 | EP | 2881917 A1 | 10-06-2015 |
| | | | ES | 2445490 A1 | 03-03-2014 |
| | | | US | 2015222875 A1 | 06-08-2015 |
| | | | WO | 2014020202 A1 | 06-02-2014 |

EPO FORM P0459

For more details about this annex : see Official Journal of the European Patent Office, No. 12/82